# EUROPÄISCHE PATENTSCHRIFT

(11) **EP 3 919 028 B1**
(45) Veröffentlichungstag und Bekanntmachung des Hinweises auf die Patenterteilung: **30.10.2024**
(21) Anmeldenummer: 20178098.8
(22) Anmeldetag: 03.06.2020
(51) Int. Cl.: A61F 2/66, A61F 2/68, A61F 2/50, A61F 2/60, A61F 2/74

(54) **ORTHESENGELENK MIT HYDRAULISCH ANPASSBARER GRUNDEINSTELLUNG SOWIE VERFAHREN ZUM HYDRAULISCHEN ANPASSEN DER GRUNDEINSTELLUNG DES ORTHESENGELENKES**
ORTHOTIC JOINT WITH HYDRAULICALLY ADAPTABLE BASIC SETTING AND METHOD FOR HYDRAULICALLY ADJUSTING THE BASIC SETTING OF THE ORTHOTIC JOINT
ARTICULATION ORTHOSIQUE À RÉGLAGE HYDRAULIQUE DE BASE AJUSTABLE AINSI QUE PROCÉDÉ D'AJUSTEMENT HYDRAULIQUE DU RÉGLAGE DE BASE DE L'ARTICULATION ORTHOSIQUE

(43) Veröffentlichungstag der Anmeldung: 08.12.2021
(73) Patentinhaber: FIOR & GENTZ Gesellschaft für Entwicklung und Vertrieb von orthopädietechnischen Systemen mbH, 21337 Lüneburg (DE)
(72) Erfinder: FIOR, Jörg, 21337 Lüneberg (DE); GENTZ, Ralf, 21337 Lüneberg (DE)
(74) Vertreter: Stork Bamberger Patentanwälte PartmbB

(56) Entgegenhaltungen:
- WO-A1-2016/100791
- WO-A2-2013/006585
- DE-B3- 102015 207 936
- DE-U1- 202010 017 697

## Beschreibung

Die Erfindung betrifft ein Orthesengelenk, insbesondere Knöchelgelenk für eine Beinorthese, umfassend ein Gelenkteil mit einer Schienenaufnahme sowie einen Fußbügel, der um eine Achse A schwenkbar mit dem Gelenkteil verbunden ist und eine dorsale Anschlagfläche und eine ventrale Anschlagfläche aufweist, wobei zu beiden Seiten der Achse A an dem Gelenkteil ein Kanal ausgebildet ist, in dem jeweils ein Funktionsmittel zum Bilden eines dorsalen Anschlags und eines ventralen Anschlag für den Fußbügel bzw. dessen Anschlagflächen am Gelenteil angeordnet ist, wobei jedem Funktionsmittel ein Stellelement mindestens zum Anpassen einer Grundeinstellung des Orthesengelenkes in Bezug auf den Verlauf einer Lotlinie einer das Orthesengelenk tragenden Person zugeordnet ist.

Des Weiteren betrifft die Erfindung ein Verfahren mindestens zum Anpassen einer Grundeinstellung eines Orthesengelenkes in Bezug auf den Verlauf einer Lotlinie einer das Orthesengelenk tragenden Person.

Solche Orthesengelenke dienen vorzugsweise in der Orthopädie zur Bildung von Unterschenkelorthesen, die auch unter "Ankle Foot Orthosis" (AFO) bekannt sind. Die Orthesengelenke dienen zur Stützung und Stabilisierung der natürlichen Gelenke. Solche Orthesengelenke kommen insbesondere bei Personen bzw. Patienten mit infantiler Cerebarparese oder vergleichbaren Erkrankungen zum Einsatz. Da jede Person eine individuelle Körperhaltung und individuelle Beeinträchtigungen und/oder Erkrankungen bzw. Behinderungen aufweist, ist auch der Verlauf der Lotlinie individuell. Letztlich verläuft die Lotlinie zwar immer vertikal nach unten. In Abhängigkeit der Körperhaltung, einer möglichen Vorlage des Körpers, einer möglichen Rücklage des Körpers, einer Schief- bzw. Schrägstellung des Beins oder anderer Einflüsse kann die Lotlinie bzw. deren Verlauf insbesondere in Bezug auf das Knöchelgelenk variieren. Die Lotlinie L kann z.B. in Bezug auf die Körpermitte durch das Knöchelgelenk laufen oder vor oder hinter dem Knöchelgelenk liegen. Als Lotlinie wird eine virtuelle Linie durch den Körpermittelpunkt beschrieben, die im Bereich des Beines bei einer stehenden Person durch die Hüfte, den Bereich des Kniegelenkes und üblicherweise kurz vor dem Knöchelgelenk verläuft und im Wesentlichen vertikal nach unten ausgerichtet ist. Das aufrechte Stehen bzw. das ausgestreckte Bein auf flachem, horizontalem Untergrund U ohne einen Absatz am Fuß stellt eine mögliche Grundeinstellung dar.

Da die die Orthese bzw. das Orthesengelenk tragende Person nicht nur aufrecht steht bzw. auf ebenem Untergrund geht, sondern sich auch bewegt, nämlich insbesondere geht bzw. läuft, ändern sich auch die Randbedingungen. Als Randbedingungen werden insbesondere das positive oder negative Gefälle des Untergrunds, auf dem die Person steht oder geht, sowie das Tragen von Schuhen mit unterschiedlicher Absatzhöhe bezeichnet, wobei ausdrücklich auch andere die Stellung des Fußes zum Unterschenkel verändernde Bedingungen als verändernde Randbedingungen angesehen werden.

Für den Tragekomfort und eine orthopädisch optimale Funktion der Orthese bzw. des Orthesengelenkes ist eine individuelle Anpassung der Orthese und des Orthesengelenkes auf unterschiedliche Randbedingungen notwendig. Ausgehend von einem flachen Untergrund, also einer horizontal ausgerichteten Trittfläche, und Schuhwerk mit flachen Absätzen bzw. barfuß ergibt sich für die Lotlinie des Beines in Bezug auf das Knöchelgelenk eine im Wesentlichen senkrecht zur Trittfläche ausgerichtete Lotlinie. Bei einem solchen Stand beträgt der Winkel α zwischen dem Fuß und dem Unterschenkel etwa 90°, wobei der Winkel α bei einer leichten Vorlage oder einer leichten Rücklage des Trägers der Orthese auch geringfügig kleiner oder größer als 90° sein kann, beispielsweise 85° oder 95°. Bei dieser Einstellung spricht man von einer Grundeinstellung oder auch Funktionsstellung der Orthese. Die Grundeinstellung kann aber auch bei anderen Randbedingungen vorgenommen werden. Aus dieser Grundeinstellung heraus erlaubt das Gelenkteil grundsätzlich eine dynamische Bewegung gegen die Funktionsmittel in Richtung einer Dorsalextension und einer Plantarflexion.

Der Komfort der Orthese und/oder deren Funktionalität ändert sich jedoch mit der Veränderung der Randbedingungen. Eine positive (z.B. beim bergauf Laufen) oder negative (z.B. beim bergab Laufen) Steigung und/oder das Tragen von Schuhwerk ohne Absatz oder mit kleinem oder großem Absatz führen dazu, dass sich der Winkel α zwischen dem Fuß und dem Unterschenkel verändert, was Auswirkungen auf die Lotlinie hat. Die verläuft zwar weiterhin vertikal nach unten, weist aber bezogen auf den Untergrund einen veränderten Winkel auf, wodurch sich die Kräfte innerhalb des Gelenkteils und insbesondere an den Anschlägen der Funktionsmittel verändern bzw. verschieben. Entsprechend ist das Orthesengelenk an die veränderten Randbedingungen anzupassen, um bei veränderten Randbedingungen eine vergleichbare Stabilität und einen entsprechenden Tragekomfort zur Verfügung stellen zu können.

Bei bisher bekannten Orthesengelenken gibt es letztlich nur eine einmalig von einer Fachperson eingestellte Grundeinstellung. Diese wird üblicherweise für eine stehende Person auf ebenem (also im Wesentlichen horizontal gerichteten) Untergrund barfuß oder mit absatzlosem Schuh eingestellt. Dazu betätigt die Fachperson Einstellschrauben als Stellelement für die Funktionsmittel, um das Orthesengelenk hinsichtlich der Anschläge und/oder der Bewegungsfreiheit des Orthesengelenkes auf die jeweilige Lotlinie bzw. deren Verlauf der das Orthesengelenk tragenden Person einzustellen bzw. anzupassen. Letztlich stellt diese eingestellte Grundeinstellung für wechselnde Randbedingungen (bergab, bergauf, Schuhwerk mit oder ohne Absatz) stets nur eine Kompromisslösung dar, da die Grundeinstellung für alle Positionen des Fußes zum Unterschenkel, also für alle Winkel α insbesondre zwischen120° und 60°, gleich ist. Eine individuelle Anpassung im Alltag ist entsprechend nicht praktikabel und nur durch eine Fachperson ausführbar. Solches Orthesengelenk ist bekannt zum Beispiel aus dem Dokument DE202010017697U1. DE202010017697U1 beschreibt ein Orthesengelenk gemäß dem Oberbegriff des Anspruchs 1.

Der Erfindung liegt somit die Aufgabe zugrunde, ein Orthesengelenk zu schaffen, das in seiner Grundeinstellung schnell und einfach durch die die Orthese tragende Person selbst an unterschiedliche Randbedingungen anpassbar ist. Der Erfindung liegt des Weiteren die Aufgabe zugrunde, ein entsprechendes Verfahren vorzuschlagen.

Diese Aufgabe wird durch ein Orthesengelenk der eingangs genannten Art dadurch gelöst, dass das oder jedes Stellelement als Hydraulikeinheit ausgebildet ist, die dem Gelenkteil zugeordnet ist und auf beide Funktionsmittel wirkt. Die oder jede Hydraulikeinheit ermöglicht das Anpassen des Orthesengelenkes auf besonders einfache und schnelle Weise. Durch die Hydraulikeinheit kann die das Orthesengelenk tragende Person selbst schnell und situativ auf eine veränderte Randbedingung, beispielsweise Veränderung der Steigung des Untergrunds, reagieren, indem das Orthesengelenk mittels der Hydraulikeinheit auf eine veränderte Ausrichtung zwischen Lotlinie einerseits und Untergrund andererseits anpassbar ist. Bei einem ebenen Untergrund steht die Lotlinie im Wesentlichen senkrecht zu der vom Untergrund aufgespannten Ebene. Einfach ausgedrückt stehen Fuß und Unterschenkel etwa in einem rechten Winkel zueinander. Bei einer positiven Steigung des Untergrunds (bergauf) verkleinert sich der Winkel (< 90°), bei einer negativen Steigung (bergab) vergrößert sich der Winkel (>90°). Entsprechend verhält es sich, wenn die Person einen Schuh mit flachem Absatz oder hohem Absatz trägt. Um die Funktionalität unabhängig von den jeweiligen Randbedingungen gleichwertig zu erhalten, ist die Anpassung der Grundeinstellung auf die Veränderung des Winkels zwischen Unterschenkel und Fuß erforderlich. Dadurch, dass die oder jede Hydraulikeinheit auf beide Funktionsmittel wirkt, ist für die die Orthese tragende Person selbst eine optimierte Anpassung zur Erhöhung des Tragekomforts und zur Verbessrung der Funktionalität an veränderte Randbedingung gewährleistet. Damit erhöhen sich die Trageoptionen für die jeweilige Person erheblich, da auf einen aufwendigen und vor allem nicht immer möglichen Eingriff durch eine Fachperson verzichtet werden kann. Es kann eine gemeinsame Hydraulikeinheit vorgesehen sein. Optional können aber auch zwei miteinander in Wirkverbindung stehende Hydraulikeinheiten vorgesehen sein. Entscheidend ist, dass die Funktionsmittel durch die oder jede Hydraulikeinheit in Anschlag mit den Anschlagflächen des Fußbügels bringbar sind.

In einer zweckmäßigen Weiterbildung der Erfindung umfasst jedes Funktionsmittel mindestens einen in dem Kanal geführten Anschlagbolzen, der mittels der mindestens einen Hydraulikeinheit gegen die Anschlagflächen des Fußbügels drückbar ist. In Abhängig der Größe des hydraulischen Drucks, der auf die Anschlagbolzen aufgebracht wird, kann eine vollständige Blockade des Orthesengelenkes erreicht werden, indem die Anschlagbolzen fest gegen die Anschlagflächen des Fußbügels gepresst werden, derart, dass diese durch die Fußkraft nicht überwunden werden kann. Bei geringerem hydraulischem Druck ist eine dynamische Bewegung in Richtung Dorsalextension und Plantarflexion gewährleistet. Als Anschlagbolzen können letztlich alle einen Anschlag bildenden Elemente eingesetzt werden, also z.B. Stifte, Stäbe, Stangen und dergleichen sowie andere einen Anschlag definierenden Komponenten. Die Hydraulikeinheit selbst kann das Funktionsmittel bilden, derart, dass diese direkt auf die Anschlagflächen des Fußbügels wirkt.

Vorteilhafterweise ist dem Anschlagbolzen mindestens ein Federelement zugeordnet. Durch das oder jedes Federelement, das einteilig oder mehrteilig ausgebildet und über den Anschlagbolzen gesteckt sein oder diesen als Funktionsmittel sogar ersetzen kann, ist die dynamische Bewegung zur Erhöhung der Stabilität des Orthesengelenkes gegen die Federkraft gewährleistet. Die Größe der Federkraft hat unmittelbar Einfluss auf die Bewegungsfreiheit des Orthesengelenkes. Sehr hohe Federkräfte führen dazu, dass diese beim Nutzen des Orthesengelenkes nicht überwunden werden könne, was einer Blockade gleichkommt.

Besonders bevorzugt ist das Federelement als austauschbare Tellerfederanordnung ausgebildet. Die Tellerfederanordnung kann aus einem oder mehreren Tellerfederpaketen bestehen, wobei jedes Tellerfederpaket wiederum aus mehreren Tellerfedern bestehen kann. Tellerfedern zeichnen sich dadurch aus, dass bei sehr kurzen Federwegen hohe Federkräfte erzeugt werden, wodurch die Stabilität in der jeweiligen Grundeinstellung maximal unterstützt wird.

Eine vorteilhafte Weiterbildung ist dadurch gekennzeichnet, dass die Hydraulikeinheit zwei Hydraulikzylinder umfasst, wobei jedem Funktionsmittel ein Hydraulikzylinder zugeordnet ist. Dadurch ist eine gleichmäßige und ausgewogene Anpassung der Grundeinstellung sichergestellt. Vorzugsweise sind die Hydraulikzylinder als einfach wirkende Hydraulikzylinder mit nur einer Kolbenfläche, die mit dem Hydraulikmedium beaufschlagt wird, ausgebildet. Solche Hydraulikzylinder weisen eine aktive Bewegungsrichtung auf. Die Rückbewegung kann dann z.B. durch das Funktionsmittel, also insbesondere die Federkraft desselben, erfolgen. Optional sind aber auch doppelt wirkende Zylinder mit gegenüberliegenden Kolbenflächen einsetzbar, die zwei aktive Bewegungsrichtungen aufweisen.

Eine besonders bevorzugte Ausführungsform sieht vor, dass in jedem Kanal ein Hydraulikzylinder ausgebildet ist, wobei ein das Hydraulikmedium aufnehmender Raum zwischen dem Funktionsmittel und einem den Kanal abschließenden Deckelelement ausgebildet ist und sowohl das Funktionsmittel als auch das Deckelelement dichtend gegenüber einer Innenwand des Kanals ausgebildet sind. Der Kanal bildet quasi das Gehäuse des Hydraulikzylinders, in dem das Funktionsmittel bzw. der Anschlagbolzen als Kolbenstange fungiert, wobei das freie Ende des Anschlagbolzens den Anschlag für die Anschlagflächen des Fußteils bildet. Der Raum zur Aufnahme des Hydraulikmediums ist gegenüber der Umgebung abgedichtet. Dazu sind das Deckelelement und das Funktionsmittel z.B. mit Ringdichtungen versehen, die gegen die Innenwand des Kanals dichten. Andere Dichtungsanordnungen oder Dichtungslösungen sind aber ebenfalls einsetzbar.

In einer zweckmäßigen Weiterbildung ist das Deckelelement jeweils als Einstellschraube ausgebildet. In dieser Ausführung ist eine zusätzliche Einstellmöglichkeit geschaffen, eine Anpassung der Grundeinstellung, insbesondere durch eine Fachperson, ergänzend oder unterstützend auszuführen. Insbesondere lässt sich durch eine solche Einstellschraube die Größe des Raums zur Aufnahme des Hydraulikmediums verändern, um die Druckverhältnisse innerhalb des Raumes einzustellen.

In einer besonders bevorzugten Ausführungsform sind die beiden Hydraulikzylinder über einen Kanal miteinander verbunden. Genauer verbindet der Kanal die beiden mit Hydraulikmedium gefüllten Räume, um einen Ausgleich zwischen den Hydraulikzylindern und damit zwischen den Funktionsmitteln zu schaffen.

Vorteilhafterweise ist in dem Kanal mindestens ein Stellventil angeordnet, das mindestens zum Öffnen und Schließen des Kanals ausgebildet und eingerichtet ist. Dadurch ist der Austausch des Hydraulikmediums zum Verändern der Größe des Raums für das Hydraulikmedium besonders einfach gewährleistet. Alleine das Öffnen des Stellventils unter den veränderten Randbedingungen führt zu einer schnellen und präzisen Anpassung des Orthesengelenkes an die veränderten Randbedingungen. Nach dem Schließen des oder jedes Stellventils unter den veränderten Randbedingungen ist das Orthesengelenk an die veränderten Randbedingungen angepasst, wobei die Funktionalität der dynamischen Bewegung gegen die Federkraft in der angepassten Grundeinstellung vollständig und gleichwertig gegeben ist. Die Anpassung kann also unter Verzicht auf eine fachkundige Mitwirkung von jeder das Orthesengelenk tragenden Person selbst und störungsfrei erfolgen.

Vorzugsweise sind in dem Kanal zwei Stellventile angeordnet, die in Reihe geschaltet sind. Dadurch lässt sich die Anpassung noch genauer und dosierter ausführen. Die Anzahl und Anordnung der Stellventile und deren Ausbildung kann variieren.

Die Hydraulikeinheit bzw. genauer das oder jedes Stellventil ist einfach manuell betätigbar. In einer weiteren Ausführungsform ist die Hydraulikeinheit mit einer Steuerungseinrichtung verbunden, derart, dass das Anpassen der Grundeinstellung an wechselnde Randbedingungen, wie z.B. abfallender oder ansteigender Untergrund oder das Wechseln von Schuhen mit Absatz auf Schuhe ohne Absatz oder umgekehrt, automatisiert mittels der Steuerungseinrichtung ausführbar ist.

Besonders bevorzugt ist das Orthesengelenk ein modulares Systemgelenk. Dadurch können einzelne Komponenten, wie z.B. die Federanordnung, zu einer noch besseren Anpassung an unterschiedliche Randbedingungen ausgetauscht werden.

Die Aufgabe wird auch durch ein Verfahren gelöst, das durch folgende Schritte gekennzeichnet ist: initiales Einstellen des Orthesengelenkes auf die Grundeinstellung der jeweiligen Person, insbesondere bei einer vorgegebenen Randbedingung mit definierter Absatzhöhe eines ausgewählten Schuhs auf horizontalem Untergrund, Verändern der Randbedingungen, insbesondere durch bergauf oder bergab stehen und/oder gehen und/oder durch Wechsel der Absatzhöhe des Schuhs, durch die das Orthesengelenk tragenden Person, Anpassen der Grundeinstellung des Orthesengelenkes durch Betätigen mindestens einer auf Funktionsmittel des Orthesengelenkes wirkenden Hydraulikeinheit, insbesondere durch Öffnen mindestens eines Stellventils, das in einem zwei Hydraulikzylinder der Hydraulikeinheit verbindenden Kanal angeordnet ist, und Schließen des oder jedes Stellventils in der Stellung der veränderten Randbedingungen. Das initiale Einstellen der Grundeinstellung, die auch als Referenzstellung bezeichnet werden kann, ist letztlich in jeder Position und unter beliebigen Randbedingungen möglich. Bevorzugt erfolgt das Einstellen jedoch barfuß oder in Schuhen ohne oder mit flachem Absatz stehend auf horizontalem Untergrund U. Im alltäglichen und bestimmungsgemäßen Gebrauch des Orthesengelenkes kommt es durch unebenen Untergrund in Folge von positiven oder negativen Steigungen und/oder bei einem Schuhwechsel situativ zu veränderten Randbedingungen, auf die die Person selbst reagieren kann.

In einer vorteilhaften Weiterbildung des Verfahrens wird mindestens das Anpassen des Orthesengelenkes an veränderte Randbedingungen wahlweise situativ durch die das Orthesengelenk tragende Person selbst und/oder durch den Einsatz einer Steuerungseinrichtung mindestens teilweise automatisch ausgeführt.

Besonders bevorzugt wird das Verfahren mit einem Orthesengelenk nach einem oder mehreren der Ansprüche 1 bis 12 ausgeführt.

Die sich aus dem erfindungsgemäßen Verfahren ergebenden Vorteile wurden bereits im Zusammenhang mit dem Orthesengelenk ausführlich beschrieben, weshalb zur Vermeidung von Wiederholungen auf die entsprechenden Passagen verwiesen wird.

Weitere zweckmäßige und/oder vorteilhafte Merkmale und Weiterbildungen zum Orthesengelenk sowie zum Verfahren ergeben sich aus den Unteransprüchen und der Beschreibung. Besonders bevorzugte Ausführungsformen des Orthesengelenkes sowie das Verfahren werden anhand der beigefügten Zeichnung näher erläutert. In der Zeichnung zeigt:
- Fig. 1: eine erste Ausführungsform des erfindungsgemäßen Orthesengelenkes,
- Fig. 2: eine vereinfachte Darstellung einer weiteren Ausführungsform des Orthesengelenkes,

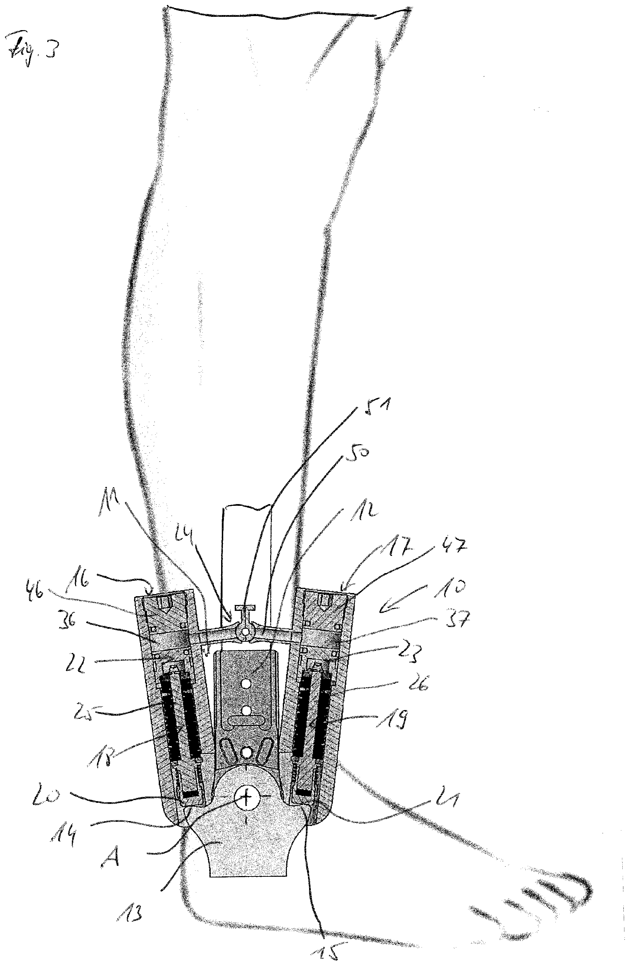

- Fig. 3: eine schematische Darstellung eines an einem Knöchel angelegten Orthesengelenkes gemäß Figur 1, und
- Fig. 4a bis e: schematische Darstellungen zur Erläuterung der Lotlinie anhand unterschiedlicher Randbedingungen.

Die Erfindung befasst sich mit einem Orthesengelenk, das zum Stützen und/oder Stabilisieren natürlicher Gelenke ausgebildet und eingerichtet ist. Beispielhaft wird die Erfindung anhand einer Knöchelgelenkorthese beschrieben. Das der Erfindung zugrundeliegende Prinzip der hydraulischen Anpassung eines Orthesengelenkes ausgehend von einer Grundeinstellung an unterschiedliche Randbedingungen ist jedoch entsprechend auch für andere Orthesengelenke, beispielsweise Armorthesen, einsetzbar.

Das in der Zeichnung dargestellte Orthesengelenk 10 ist ein Knöchelgelenk für eine Beinorthese und umfasst ein Gelenkteil 11 mit einer Schienenaufnahme 12 sowie einen Fußbügel 13, der um eine Achse A schwenkbar mit dem Gelenkteil 11 verbunden ist und eine dorsale Anschlagfläche 14 und eine ventrale Anschlagfläche 15 aufweist, wobei zu beiden Seiten der Achse A an dem Gelenkteil 11 ein Kanal 16, 17 ausgebildet ist, in dem jeweils ein Funktionsmittel 18, 19 zum Bilden eines dorsalen Anschlags 20 und eines ventralen Anschlags 21 für den Fußbügel 13 bzw. dessen Anschlagflächen 14, 15 am Gelenteil 11 angeordnet ist, wobei jedem Funktionsmittel 18, 19 ein Stellelement 22, 23 mindestens zum Anpassen einer Grundeinstellung des Orthesengelenkes 10 in Bezug auf den Verlauf einer Lotlinie einer das Orthesengelenk 10 tragenden Person zugeordnet ist. Optional kann das oder jedes Stellelement 22, 23 zusätzlich auch zum Anpassen der Beweglichkeit bzw. des Bewegungsspielraums des Orthesengelenkes 10 dienen.

Dieses Orthesengelenk 10 zeichnet sich erfindungsgemäß dadurch aus, dass das oder jedes Stellelement 22, 23 als Hydraulikeinheit 24 ausgebildet ist, die dem Gelenkteil 11 zugeordnet ist und auf beide Funktionsmittel 18, 19 wirkt. Sowohl die initiale Einstellung der Grundeinstellung durch eine Fachperson oder die das Orthesengelenk 10 tragende Person selbst als auch das situative Anpassen der Grundeinstellung an sich verändernde Randbedingungen durch die das Orthesengelenk 10 tragende Person selbst ist ohne Hilfsmittel schnell und einfach ausführbar. Letztlich ermöglicht die auf beide Funktionsmittel 18, 19 wirkende Hydraulikeinheit 24 ausgehend von der initialen Einstellung der Grundeinstellung eine schnelle und präzise Anpassung auf die sich insbesondere durch eine (positive oder negative) Steigung des Untergrunds U und/oder Absätze am Schuh ergebende Winkeländerung des Winkels α zwischen Fuß und Unterschenkel, um eine optimale Stützung der das Orthesengelenk 10 tragenden Person zu ermöglichen. Die oder jede Hydraulikeinheit 24 selbst kann auch das Funktionsmittel 18, 19 bilden, das gegen die Anschlagflächen 14, 15 wirkt.

Die im Folgenden beschriebenen Merkmale und Weiterbildungen stellen für sich betrachtet und in Kombination miteinander bevorzugte Ausführungsformen dar. Es wird ausdrücklich darauf hingewiesen, dass Merkmale, die in den Ansprüchen und/oder der Beschreibung und/oder der Zeichnung zusammengefasst oder in einer gemeinsamen Ausführungsform beschrieben sind, auch funktional eigenständig das weiter oben beschriebene Orthesengelenk 10 weiterbilden können.

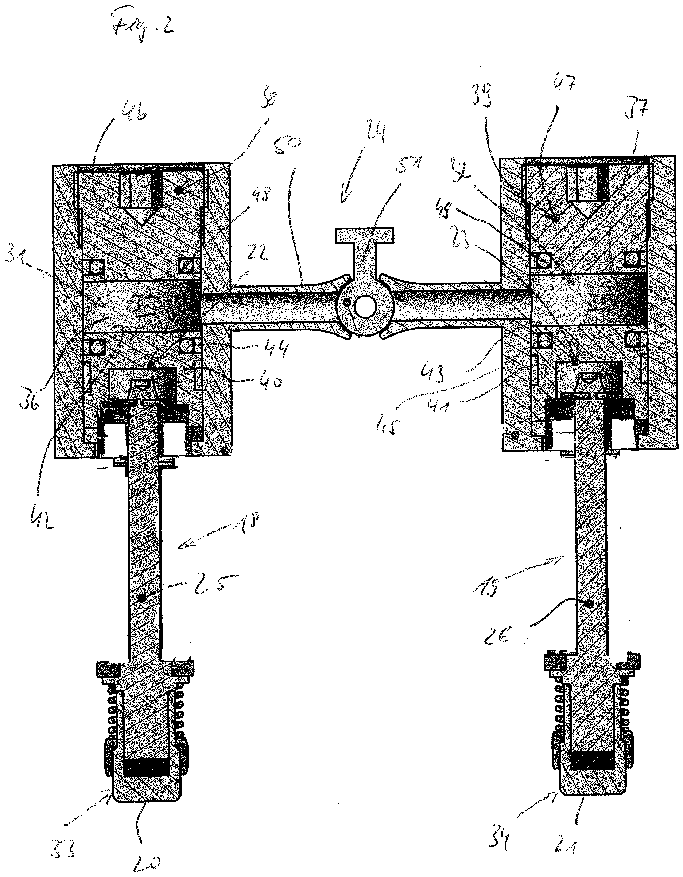

Die Funktionsmittel 18, 19 können unterschiedlich ausgebildet und wahlweise zum vollständigen Blockieren des Orthesengelenkes 10 oder zum Erlauben einer dynamischen Bewegung in Richtung Dorsalextension oder Plantarflexion eingerichtet sein. In einer Ausführungsform (siehe insbesondere Figur 2) umfasst jedes Funktionsmittel 18, 19 mindestens einen in dem Kanal 16, 17 geführten Anschlagbolzen 25, 26, der mittels der mindestens einen Hydraulikeinheit 24 gegen die Anschlagflächen 14, 15 des Fußbügels 13 drückbar ist. Der Anschlagbolzen 25, 26 kann ein einfacher Stift sein. Andere Ausführungen des Anschlagbolzens 25, 26 sind aber ebenfalls denkbar, solange ein Anschlag 20, 21 ausgebildet ist. In der dargestellten Ausführungsform gemäß Figur 2 ist am freien Ende des Anschlagbolzens 25, 26 ein federnd gelagerter Aufsatz 33, 34 angeordnet, der den Anschlag 20, 21 bildet.

Vorzugsweise ist dem Anschlagbolzen 25, 26 mindestens ein Federelement 27, 28 zugeordnet. Mit dieser Ausbildung ist aus der Grundeinstellung heraus eine dynamische Bewegung gegen die Federkräfte der Federelemente 27, 28 in Richtung Dorsalextension und Plantarflexion gewährleistet, um sich dem natürlichen Gangbild anzunähern bzw. bei maximaler Stabilität die Beweglichkeit zu steigern. Der Grad der Bewegungsfreiheit hängt wesentlich von der Federspannung und/oder dem Druck des Hydraulikmediums ab. Optional und bevorzugt ist eine Bewegungsfreiheit zwischen den beiden Anschlägen 20, 21 von +/- 17° eingestellt. Der Winkel, innerhalb dem sich die Bewegungsfreiheit ergibt, kann selbstverständlich auch einen anderen Wert aufweisen.

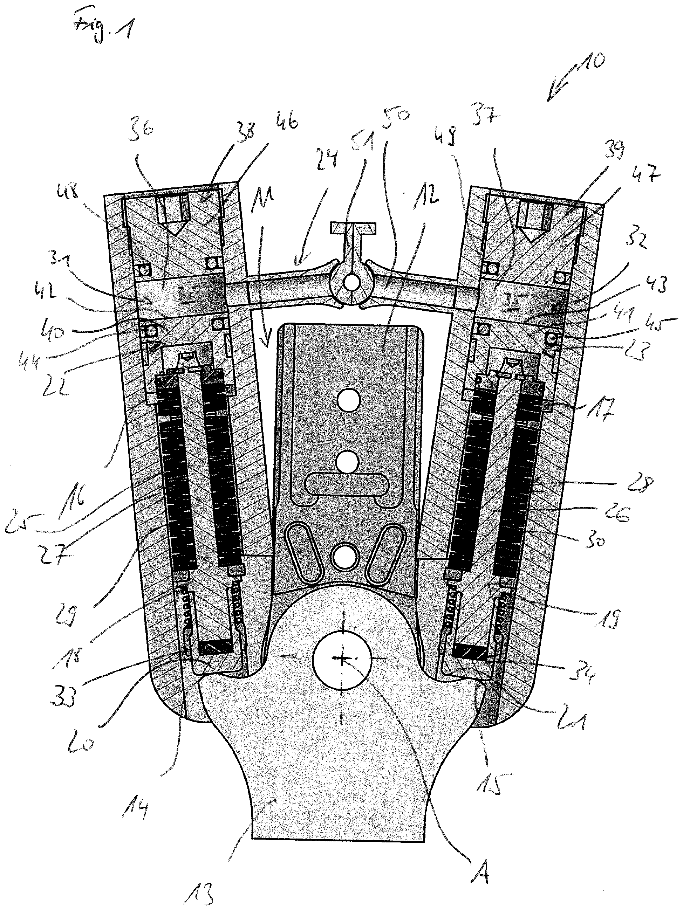

Als Federelemente 27, 28 können z.B. einfache Spiralfedern eingesetzt werden. Die Federelemente 27, 28 selbst können auch das Funktionsmittel 18, 19 darstellen bzw. bilden. Besonders bevorzugt und beispielhaft insbesondere der Figur 1 zu entnehmen ist jedes Federelement 27, 28 als austauschbare Tellerfederanordnung 29, 30 ausgebildet. Die aus einem oder mehreren Tellerfederpakten, die jeweils aus mehreren Tellerfedern bestehen können, gebildeten Tellerfederanordnungen 29, 30 sind auf den Anschlagbolzen 25, 26 aufgesteckt. Andere Federkomponenten und Federanordnungen sind aber ebenfalls einsetzbar.

Wie erwähnt, ist das oder jedes Stellelement 22, 23 als Hydraulikeinheit 24 ausgebildet. Es besteht die Möglichkeit, dass jedem Funktionsmittel 18, 19 eine eigene separate Hydraulikeinheit zugeordnet ist. Vorzugsweise ist jedoch eine gemeinsame Hydraulikeinheit 24 für beide bzw. zur Betätigung beider Funktionsmittel 18, 19 vorgesehen. Diese Hydraulikeinheit 24 umfasst zwei Hydraulikzylinder 31, 32, wobei jedem Funktionsmittel 18, 19 ein Hydraulikzylinder 31, 32 zugeordnet ist. Bevorzugt ist dazu in jedem Kanal 16, 17 ein Hydraulikzylinder 31, 32 ausgebildet. Der Kanal 16, 17 bildet dabei das Gehäuse des Hydraulikzylinders 31, 32, wobei ein das Hydraulikmedium 35 aufnehmender Raum 36, 37 zwischen dem Funktionsmittel 18, 19 und einem den Kanal 16, 17 abschließenden Deckelelement 38, 39 ausgebildet ist und sowohl das Funktionsmittel 18, 19 als auch das Deckelelement 38, 39 dichtend gegenüber einer Innenwand des Kanals 16, 17 ausgebildet sind.

Ein Kolbenelement 40, 41 bildet eine dem Raum 36, 37 zugewandte Kolbenfläche 42, 43. Das Kolbenelement ist gleitend innerhalb des Kanals 16, 17 angeordnet und z.B. mittels eines Dichtrings 44, 45 zur Kanalwand hin gedichtet. An dem Kolbenelement 40, 41 ist das Funktionsmittel 18, 19 angeordnet. Durch den hydraulischen Druck auf die Kolbenfläche 42, 43 ist das Funktionsmittel 18, 19 mit seinem Anschlag 20, 21 aktiv in Richtung der Anschlagflächen 14, 15 des Fußbügels 13 gehalten. Die Federkraft der Tellerfederanordnung 29, 30 bildet die Rückstellkraft für das Kolbenelement 40, 41, gegen die eine dynamische Bewegung in Richtung Dorsalextension und Plantarflexion gegeben ist. Das Deckelelement 38, 39 kann eine einfache Kappe sein, die aufgesteckt oder aufgeschraubt ist. Vorzugsweise ist das Deckelelement 38, 39 jeweils als Einstellschraube 46, 47 ausgebildet. Diese Einstellschraube 46, 47 weist einen Dichtring 48, 49 auf, mittels dem die Einstellschraube zur Kanalwand hin gedichtet ist. Anstelle der Dichtringe 44, 45 und 48, 49 können auch andere Dichtelemente eingesetzt werden.

Jeder Hydraulikzylinder 31, 32 kann separat und unabhängig zum jeweils anderen Hydraulikzylinder 31, 32 ausgebildet und eingerichtet sein, wobei die beiden Hydraulikzylinder 31, 32 dann hinsichtlich ihrer Wirkungsweise und Funktionalität aufeinander abgestimmt sind. Vorzugsweise sind die beiden Hydraulikzylinder 31, 32 jedoch über einen Kanal 50 miteinander verbunden. Der Kanal 50 verbindet die beiden das Hydraulikmedium 35 beinhaltenden Räume 36, 37, so dass ein Austausch des Hydraulikmediums 35 zwischen den Räumen 36, 37 zur Veränderung deren Größe gewährleistet ist. Vorzugsweise ist in dem Kanal 50 mindestens ein Stellventil 51 angeordnet ist, das mindestens zum Öffnen und Schließen des Kanals 50 ausgebildet und eingerichtet ist. Dadurch kann beim Öffnen des Stellventils 51 I ein Austausch des Hydraulikmediums 35 stattfinden, um das Orthesengelenk 10 bzw. dessen Grundeinstellung, vorzugsweise bedingt durch eine Winkelveränderung zwischen Fuß und Unterschenkel - an geänderte Randbedingungen anzupassen um diese angepasste Grundeinstellung dann durch Schließen des Stellventils 51 zu sichern und zu halten. In anderen nicht explizit dargestellten Ausführungsformen sind in dem Kanal 50 zwei Stellventile 51 angeordnet, die in Reihe geschaltet sind. Die Anzahl der Stellventile 51, deren Anordnung und Ausbildung kann variieren. Das oder jedes Stellventil 51 ist manuell oder automatisiert betätigbar.

Zum automatisierten Betätigen ist die Hydraulikeinheit 24 mit einer nicht explizit dargestellten Steuerungseinrichtung verbunden, derart, dass das Anpassen der Grundeinstellung an wechselnde Randbedingungen, wie z.B. abfallender oder ansteigender Untergrund oder das Wechseln von Schuhen mit Absatz auf Schuhe ohne Absatz oder umgekehrt automatisiert mittels der Steuerungseinrichtung ausführbar ist.

Besonders bevorzugt ist das Orthesengelenk 10 ein modulares Systemgelenk.

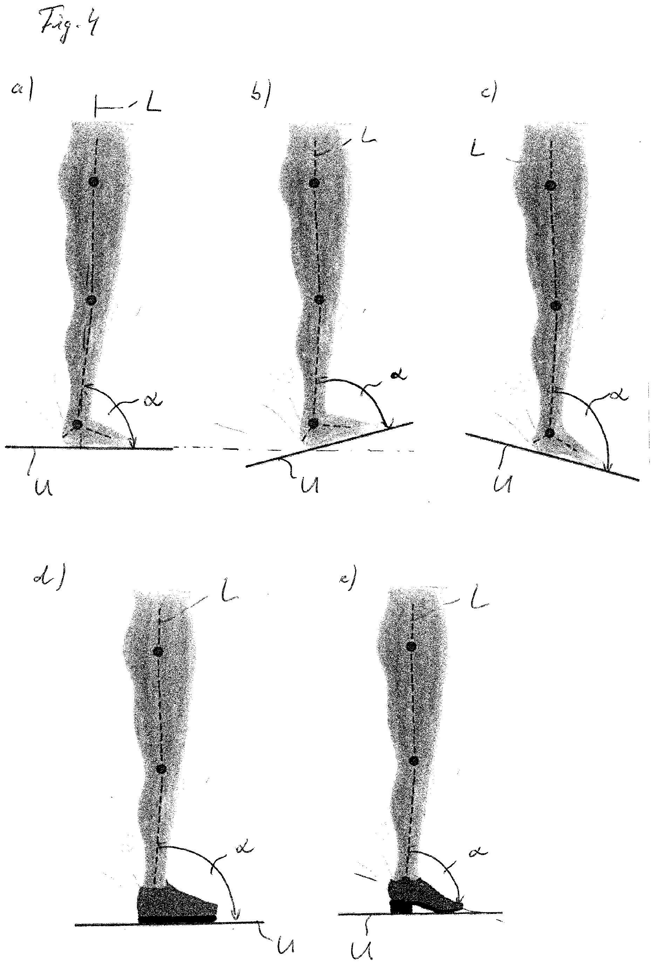

Im Folgenden wird das erfindungsgemäße Verfahren anhand der Zeichnung näher erläutert:
Das Verfahren zum Anpassen der Grundeinstellung in Bezug auf den Verlauf einer Lotlinie L einer das Orthesengelenk 10 tragenden Person erfolgt bei angelegtem Orthesengelenk 10. Da jede Person einen individuellen Verlauf der Lotlinie L aufweist, ist die Grundeinstellung auch individuell darauf einzustellen. Der Verlauf der Lotlinie L, die grundsätzlich vertikal nach unten gerichtet verläuft, kann - z.B. durch Vorlage oder Rücklage der Person, Schrägstellung des Beins oder anderer Gründe - mal durch das Knöchelgelenk verlaufen, mal knapp davor oder weiter davor, mal knapp dahinter oder weiter dahinter liegen. In den Figuren 4 a bis e sind verschiedene Randbedingungen dargestellt. In den Figuren 4 a und d steht die Lotlinie L etwa senkrecht zum Untergrund U, der Winkel α beträgt etwa 90°. In der Figur 4b steht die Person bergauf, weshalb der Winkel α < 90° ist. In den Figuren 4 c und e ist der Winkel α > 90°, da die Person bergab steht bzw. einen Schuh mit höherem Absatz trägt.

Zunächst wird das Orthesengelenkes 10 initial auf die Grundeinstellung der jeweiligen Person eingestellt. Diese Einstellung kann auch als Referenz angesehen werden. Bevorzugt erfolgt diese Einstellung bei einer vorgegebenen Randbedingung, beispielsweise mit definierter Absatzhöhe eines ausgewählten Schuhs auf horizontalem Untergrund U (siehe z.B. Figuren 4a und 4d). Mit dieser Grundeinstellung kann die jeweilige Person das Orthesengelenk optimal bei gleichbleibenden Randbedingungen nutzen, um maximale Stabilität und Unterstützung des natürlichen Gelenkes und der gestützten Gliedmaßen zu haben. Die bestimmungsgemäße Nutzung des Orthesengelenkes 10 führt zwangsläufig zum Verändern der Randbedingungen, insbesondere durch bergauf oder bergab stehen und/oder gehen und/oder durch Wechsel der Absatzhöhe des Schuhs, durch die das Orthesengelenk 10 tragenden Person. Entsprechend erfolgt dann das Anpassen der Grundeinstellung des Orthesengelenkes 10, und zwar erfindungsgemäß durch Betätigen mindestens einer auf Funktionsmittel 18, 19 des Orthesengelenkes 10 wirkenden Hydraulikeinheit 24. Mindestens das Anpassen an die geänderten Randbedingungen erfolgt durch die das Orthesengelenk 10 tragende Person selbst. Das initiale Einstellen kann von einer Fachperson oder der das Orthesengelenk 10 tragenden Person selbst ausgeführt werden.

Die Hydraulikeinheit 24 kann auch unterschiedliche Weise auf die Funktionsmittel 18, 19, die mit Anschlägen 20, 21 gegen Anschlagflächen 14, 15 eines Fußbügels 13 des Orthesengelenkes 10 drücken, wirken. Bevorzugt erfolgt die Anpassung in einer Stellung der Person mit veränderten Randbedingungen, also z.B. in einer bergauf Stellung, bei der der Winkel α zwischen Fuß und Unterschenkel < 90° ist, durch Öffnen mindestens eines Stellventils 51, das in einem zwei Hydraulikzylinder 31, 32 der Hydraulikeinheit 24 verbindenden Kanal 50 angeordnet ist, und Schließen des oder jedes Stellventils 51 in der Stellung der veränderten Randbedingungen. Durch das Öffnen des oder jedes Stellventils 51 kann das Hydraulikmedium 35 zwischen den Hydraulikzylindern 31, 32 hin und her strömen. Durch die aufgrund der veränderten Randbedingungen veränderte Winkelstellung von Fuß zu Unterschenkel wird ein das Hydraulikmedium 35 beinhaltender Raum 36 des einen Hydraulikzylinders 31 kleiner, während ein das Hydraulikmedium 35 beinhaltender Raum 37 des anderen Hydraulikzylinders 32 größer wird oder umgekehrt. Damit ist das Orthesengelenk 10 ausgehend von der initialen Grundeinstellung an die geänderten Randbedingungen angepasst, und die Funktionalität insbesondere der dynamischen Bewegungen gegen die Funktionsmittel 18, 19 in Richtung Dorsalextension und Plantarflexion ist aus dieser angepassten Grundeinstellung heraus vollständig und gleichwertig gegeben.

Optional wird mindestens das Anpassen des Orthesengelenkes 10 an veränderte Randbedingungen wahlweise situativ durch die das Orthesengelenk 10 tragende Person selbst und/oder durch den Einsatz einer Steuerungseinrichtung mindestens teilweise automatisch ausgeführt. Neben der Anpassung der Grundeinstellung an die veränderten Randbedingungen kann mittels der Hydraulikeinheit 24 auch die Beweglichkeit des Orthesengelenkes 10 eingestellt werden. Ein hoher hydraulischer Druck kann dazu führen, dass das Orthesengelenk 10 quasi blockiert ist. Mit Reduzierung des hydraulischen Drucks wird eine Bewegbarkeit im Rahmen der Anschläge 20, 21 gegen den Fußbügel 13 ermöglicht, die auch noch durch die Federspannung der Tellerfederanordnungen 29, 30 beeinflusst wird.

Besonders bevorzugt wird das Verfahren mit einem Orthesengelenk 10 nach einem oder mehreren der Ansprüche 1 bis 12 ausgeführt.

## Patentansprüche

1. Orthesengelenk (10), insbesondere Knöchelgelenk für eine Beinorthese, umfassend ein Gelenkteil (11) mit einer Schienenaufnahme (12) sowie einen Fußbügel (13), der um eine Achse A schwenkbar mit dem Gelenkteil (11) verbunden ist und eine dorsale Anschlagfläche (14) und eine ventrale Anschlagfläche (15) aufweist, wobei zu beiden Seiten der Achse A an dem Gelenkteil (11) ein Kanal (16, 17) ausgebildet ist, in dem jeweils ein Funktionsmittel (18, 19) zum Bilden eines dorsalen Anschlags (20) und eines ventralen Anschlags (21) für den Fußbügel (13) bzw. dessen Anschlagflächen (14, 15) am Gelenteil (11) angeordnet ist, wobei jedem Funktionsmittel (18, 19) ein Stellelement (22, 23) mindestens zum Anpassen einer Grundeinstellung des Orthesengelenkes (10) in Bezug auf den Verlauf einer Lotlinie einer das Orthesengelenk (10) tragenden Person zugeordnet ist, **dadurch gekennzeichnet, dass** das oder jedes Stellelement (22, 23) als Hydraulikeinheit (24) ausgebildet ist, die dem Gelenkteil (11) zugeordnet ist und auf beide Funktionsmittel (18, 19) wirkt.

2. Orthesengelenk (10) nach Anspruch 1, **dadurch gekennzeichnet, dass** jedes Funktionsmittel (18, 19) mindestens einen in dem Kanal (16, 17) geführten Anschlagbolzen (25, 26) umfasst, der mittels der mindestens einen Hydraulikeinheit (24) gegen die Anschlagflächen (14, 15) des Fußbügels (13) drückbar ist.

3. Orthesengelenk (10) nach Anspruch 2, **dadurch gekennzeichnet, dass** dem Anschlagbolzen (25, 26) mindestens ein Federelement (27, 28) zugeordnet ist.

4. Orthesengelenk (10) nach Anspruch 3, **dadurch gekennzeichnet, dass** das Federelement (27, 28) als austauschbare Tellerfederanordnung (29, 30) ausgebildet ist.

5. Orthesengelenk (10) nach einem oder mehreren der Ansprühe 1 bis 4, **dadurch gekennzeichnet, dass** die Hydraulikeinheit (24) zwei Hydraulikzylinder (31, 32) umfasst, wobei jedem Funktionsmittel (18, 19) ein Hydraulikzylinder (31, 32) zugeordnet ist.

6. Orthesengelenk (10) nach Anspruch 5, **dadurch gekennzeichnet, dass** in jedem Kanal (16, 17) ein Hydraulikzylinder (31, 32) ausgebildet ist, wobei ein das Hydraulikmedium (35) aufnehmender Raum (36, 37) zwischen dem Funktionsmittel (18, 19) und einem den Kanal (16, 17) abschließenden Deckelelement (38, 39) ausgebildet ist und sowohl das Funktionsmittel (18, 19) als auch das Deckelelement (38, 39) dichtend gegenüber einer Innenwand des Kanals (16, 17) ausgebildet sind.

7. Orthesengelenk (10) nach Anspruch 6, **dadurch gekennzeichnet, dass** das Deckelelement (38, 39) jeweils als Einstellschraube (46, 47) ausgebildet ist.

8. Orthesengelenk (10) nach einem oder mehreren der Ansprüche 5 bis 7, **dadurch gekennzeichnet, dass** die beiden Hydraulikzylinder (31, 32) über einen Kanal (50) miteinander verbunden sind.

9. Orthesengelenk (10) nach Anspruch 8, **dadurch gekennzeichnet, dass** in dem Kanal (50) mindestens ein Stellventil (51) angeordnet ist, das mindestens zum Öffnen und Schließen des Kanals (50) ausgebildet und eingerichtet ist.

10. Orthesengelenk (10) nach Anspruch 8 oder 9, **dadurch gekennzeichnet, dass** in dem Kanal (50) zwei Stellventile (51) angeordnet sind, die in Reihe geschaltet sind.

11. Orthesengelenk (10) nach einem oder mehreren der Ansprüche 1 bis 10, **dadurch gekennzeichnet, dass** die Hydraulikeinheit (24) mit einer Steuerungseinrichtung verbunden ist, derart, dass das Anpassen der Grundeinstellung an wechselnde Randbedingungen, wie z.B. abfallender oder ansteigender Untergrund oder das Wechseln von Schuhen mit Absatz auf Schuhe ohne Absatz oder umgekehrt automatisiert mittels der Steuerungseinrichtung ausführbar ist.

12. Orthesengelenk (10) nach einem oder mehreren der Ansprüche 1 bis 11, **dadurch gekennzeichnet, dass** das Orthesengelenk (10) ein modulares Systemgelenk ist.

13. Verfahren mindestens zum Anpassen einer Grundeinstellung eines Orthesengelenkes (10) nach einem oder mehreren der Ansprüche 1-12 in Bezug auf den Verlauf einer Lotlinie einer das Orthesengelenk (10) tragenden Person, **gekennzeichnet durch** die folgenden Schritte:
- initiales Einstellen des Orthesengelenkes (10) auf die Grundeinstellung der jeweiligen Person, insbesondere bei einer vorgegebenen Randbedingung mit definierter Absatzhöhe eines ausgewählten Schuhs auf horizontalem Untergrund,
- Verändern der Randbedingungen, insbesondere durch bergauf oder bergab stehen und/oder gehen und/oder durch Wechsel der Absatzhöhe des Schuhs, durch die das Orthesengelenk (10) tragenden Person,
- Anpassen der Grundeinstellung des Orthesengelenkes (10) durch Betätigen mindestens einer auf Funktionsmittel (18, 19) des Orthesengelenkes (10) wirkenden Hydraulikeinheit (24), insbesondere durch Öffnen mindestens eines Stellventils (51), das in einem zwei Hydraulikzylinder (31, 32) der Hydraulikeinheit (24) verbindenden Kanal (50) angeordnet ist, und Schließen des oder jedes Stellventils (51) in der Stellung der veränderten Randbedingungen.

14. Verfahren nach Anspruch 13, **dadurch gekennzeichnet, dass** mindestens das Anpassen des Orthesengelenkes (10) an veränderte Randbedingungen wahlweise situativ durch die das Orthesengelenk (10) tragende Person selbst und/oder durch den Einsatz einer Steuerungseinrichtung mindestens teilweise automatisch ausgeführt wird.

15. Verfahren nach Anspruch 13 oder 14, **dadurch gekennzeichnet, dass** es mit einem Orthesengelenk (10) nach einem oder mehreren der Ansprüche 1 bis 12 ausgeführt wird.

## Claims

1. Orthotic joint (10), in particular ankle joint for a leg orthosis, comprising a joint part (11) with a rail mount (12) and a foot stirrup (13) which is connected to the joint part (11) so as to be pivotable about an axis A and presents a dorsal stop surface (14) and a ventral stop surface (15), with a channel (16, 17) being formed on both sides of the axis A on the joint part (11), in each of which a functional means (18, 19) for forming a dorsal stop (20) and a ventral stop (21) for the foot stirrup (13) or its stop surfaces (14, 15) is arranged on the joint part (11), an adjusting element (22, 23) being assigned to each functional means (18, 19) at least for adjusting a base setting of the orthotic joint (10) in relation to the course of a plumb line of a person wearing the orthotic joint (10), **characterised in that** the or each adjusting element (22, 23) is configured as a hydraulic unit (24) which is assigned to the joint part (11) and acts on both functional means (18, 19).

2. Orthotic joint (10) according to claim 1, **characterised in that** each functional means (18, 19) comprises at least one stop pin (25, 26) which is guided in the channel (16, 17) and can be pressed against the stop surfaces (14, 15) of the foot stirrup (13) by means of the at least one hydraulic unit (24).

3. Orthotic joint (10) according to claim 2, **characterised in that** at least one spring element (27, 28) is assigned to the stop pin (25, 26).

4. Orthotic joint (10) according to claim 3, **characterised in that** the spring element (27, 28) is configured as an exchangeable disc spring arrangement (29, 30).

5. Orthotic joint (10) according to one or more of claims 1 to 4, **characterised in that** the hydraulic unit (24) comprises two hydraulic cylinders (31, 32), one hydraulic cylinder (31, 32) being assigned to each functional means (18, 19).

6. Orthotic joint (10) according to claim 5, **characterised in that** a hydraulic cylinder (31, 32) is formed in each channel (16, 17), wherein a space (36, 37) receiving the hydraulic medium (35) is formed between the functional means (18, 19) and a cover element (38, 39) closing off the channel (16, 17), and both the functional means (18, 19) and the cover element (38, 39) are configured to seal against an inner wall of the channel (16, 17).

7. Orthotic joint (10) according to claim 6, **characterised in that** the cover element (38, 39) is configured in each case as an adjusting screw (46, 47).

8. Orthotic joint (10) according to one or more of claims 5 to 7, **characterised in that** the two hydraulic cylinders (31, 32) are connected to one another via a channel (50).

9. Orthotic joint (10) according to claim 8, **characterised in that** at least one control valve (51) is arranged in the channel (50) and is configured and adapted at least to open and close the channel (50).

10. Orthotic joint (10) according to claim 8 or 9, **characterised in that** two control valves (51) connected in series are arranged in the channel (50).

11. Orthotic joint (10) according to one or more of claims 1 to 10, **characterised in that** the hydraulic unit (24) is connected to a control device such that the adjustment of the base setting to changing parameters, such as a falling or rising surface or the change from shoes with heels to shoes without heels or vice versa can be carried out automatically by means of the control device.

12. Orthotic joint (10) according to one or more of claims 1 to 11, **characterised in that** the orthotic joint (10) is a modular system joint.

13. Method at least for adjusting a base setting of an orthotic joint (10) according to one or more of claims 1-12 in relation to the course of a plumb line of a person wearing the orthotic joint (10), **characterised by** the following steps:
- Initial adjustment of the orthotic joint (10) to the base setting of the person concerned, in particular for a given parameter with a defined heel height of a selected shoe on a horizontal surface.
- Changing the parameters, in particular due to the person wearing the orthotic joint (10) standing and/or walking uphill or downhill and/or changing the heel height of the shoe.
- Adjustment of the base setting of the orthotic joint (10) by actuating at least one hydraulic unit (24) acting on functional means (18, 19) of the orthotic joint (10), in particular by opening at least one control valve (51) which is arranged in a channel (50) connecting two hydraulic cylinders (31, 32) of the hydraulic unit (24), and closing the or each control valve (51) in the position of the changed parameters.

14. Method according to claim 13, **characterised in that** at least the adjustment of the orthotic joint (10) to changed parameters is optionally carried out depending on the situation by the person wearing the orthotic joint (10) themselves and/or at least partially automatically by the use of a control device.

15. Method according to claim 13 or 14, **characterised in that** it is carried out with an orthotic joint (10) according to one or more of claims 1 to 12.

## Revendications

1. Articulation orthosique (10), notamment articulation de cheville pour une orthèse de jambe, comprenant une partie d'articulation (11) avec un logement d'attelle (12) ainsi qu'un étrier de pied (13) qui est relié à la partie d'articulation (11) de manière pivotante autour d'un axe A et qui présente une surface de butée dorsale (14) et une surface de butée ventrale (15), un canal (16, 17) étant configuré des deux côtés de l'axe A sur la partie d'articulation (11), canal dans lequel est agencé respectivement un moyen fonctionnel (18, 19) pour former une butée dorsale (20) et une butée ventrale (21) pour l'étrier de pied (13) ou ses surfaces de butée (14, 15) sur la partie d'articulation (11), un élément de réglage (22, 23) étant associé à chaque moyen fonctionnel (18, 19) au moins pour ajuster un réglage de base de l'articulation orthosique (10) par rapport au tracé d'une ligne d'aplomb d'une personne portant l'articulation orthosique (10), **caractérisée en ce que** le ou chaque élément de réglage (22, 23) est configuré sous forme d'unité hydraulique (24) qui est associée à la partie d'articulation (11) et agit sur les deux moyens fonctionnels (18, 19).

2. Articulation orthosique (10) selon la revendication 1, **caractérisée en ce que** chaque moyen fonctionnel (18, 19) comprend au moins un boulon de butée (25, 26) guidé dans le canal (16, 17), qui peut être pressé contre les surfaces de butée (14, 15) de l'étrier de pied (13) au moyen de l'au moins une unité hydraulique (24).

3. Articulation orthosique (10) selon la revendication 2, **caractérisée en ce qu'**au moins un élément de ressort (27, 28) est associé au boulon de butée (25, 26).

4. Articulation orthosique (10) selon la revendication 3, **caractérisée en ce que** l'élément de ressort (27, 28) est configuré sous forme d'agencement de rondelle-ressort (29, 30) interchangeable.

5. Articulation orthosique (10) selon une ou plusieurs des revendications 1 à 4, **caractérisée en ce que** l'unité hydraulique (24) comprend deux cylindres hydrauliques (31, 32), un cylindre hydraulique (31, 32) étant associé à chaque moyen fonctionnel (18, 19).

6. Articulation orthosique (10) selon la revendication 5, **caractérisée en ce qu'**un cylindre hydraulique (31, 32) est configuré dans chaque canal (16, 17), un espace (36, 37) recevant le fluide hydraulique (35) étant configuré entre le moyen fonctionnel (18, 19) et un élément de couvercle (38, 39) fermant le canal (16, 17), et aussi bien le moyen fonctionnel (18, 19) que l'élément de couvercle (38, 39) étant configurés sous forme étanche par rapport à une paroi intérieure du canal (16, 17).

7. Articulation orthosique (10) selon la revendication 6, **caractérisée en ce que** l'élément de couvercle (38, 39) est respectivement configuré sous forme de vis de réglage (46, 47).

8. Articulation orthosique (10) selon une ou plusieurs des revendications 5 à 7, **caractérisée en ce que** les deux cylindres hydrauliques (31, 32) sont reliés entre eux par l'intermédiaire d'un canal (50).

9. Articulation orthosique (10) selon la revendication 8, **caractérisée en ce que** dans le canal (50) est agencée au moins une soupape de réglage (51) qui est configurée et adaptée au moins pour ouvrir et fermer le canal (50).

10. Articulation orthosique (10) selon la revendication 8 ou 9, **caractérisée en ce que** deux soupapes de réglage (51) sont agencées dans le canal (50), qui sont montées en série.

11. Articulation orthosique (10) selon une ou plusieurs des revendications 1 à 10, **caractérisée en ce que** l'unité hydraulique (24) est reliée à un dispositif de commande de telle sorte que l'ajustement du réglage de base à des conditions limites variables, comme p. ex. un sol en pente ou en montée ou le passage de chaussures avec talon à des chaussures sans talon ou inversement, peut être effectué de manière automatisée au moyen du dispositif de commande.

12. Articulation orthosique (10) selon une ou plusieurs des revendications 1 à 11, **caractérisée en ce que** l'articulation orthosique (10) est une articulation de système modulaire.

13. Procédé au moins pour adapter un réglage de base d'une articulation orthosique (10) selon une ou plusieurs des revendications 1 à 12 par rapport au tracé d'une ligne d'aplomb d'une personne portant l'articulation orthosique (10), **caractérisé par** les étapes suivantes :
- le réglage initial de l'articulation orthosique (10) sur le réglage de base de la personne concernée, notamment dans le cas d'une condition limite prédéfinie avec une hauteur de talon définie d'une chaussure choisie sur un sol horizontal,
- la modification des conditions limites, notamment en se tenant debout et/ou en marchant en montée ou en descente et/ou en changeant la hauteur de talon de la chaussure, par la personne portant l'articulation orthosique (10),
- l'ajustement du réglage de base de l'articulation orthosique (10) par actionnement d'au moins une unité hydraulique (24) agissant sur des moyens fonctionnels (18, 19) de l'articulation orthosique (10), notamment par ouverture d'au moins une soupape de réglage (51) agencée dans un canal (50) reliant deux cylindres hydrauliques (31, 32) de l'unité hydraulique (24), et fermeture de la ou de chaque soupape de réglage (51) dans la position des conditions limites modifiées.

14. Procédé selon la revendication 13, **caractérisé en ce qu'**au moins l'ajustement de l'articulation orthosique (10) à des conditions limites modifiées est effectué au choix en fonction de la situation par la personne portant l'articulation orthosique (10) elle-même et/ou de manière au moins partiellement automatisée par l'utilisation d'un dispositif de commande.

15. Procédé selon la revendication 13 ou 14, **caractérisé en ce qu'**il est réalisé avec une articulation orthosique (10) selon une ou plusieurs des revendications 1 à 12.
